# FASCICULE DE BREVET EUROPEEN

(11) **EP 3 176 668 B1**
(45) Date de publication et mention de la délivrance du brevet: **17.06.2020**
(21) Numéro de dépôt: 16306540.2
(22) Date de dépôt: 23.11.2016
(51) Int. Cl.: C07C 2/32, G05D 21/02, B01J 31/02, B01J 31/14, G05B 13/04

(54) **UTILISATION D'UN CONTRÔLEUR MULTIVARIABLE AVANCÉ POUR LE CONTRÔLE DES UNITÉS ALPHABUTOL**
VERWENDUNG EINES FORTSCHRITTLICHEN MULTIVARIABLEN CONTROLLERS FÜR DIE KONTROLLE VON ALPHABUTOL-EINHEITEN
USE OF AN ADVANCED MULTIVARIABLE CONTROLLER TO CONTROL ALPHABUTOL UNITS

(30) Priorité: 03.12.2015 FR 1561827
(43) Date de publication de la demande: 07.06.2017
(73) Titulaire: AXENS, 92508 Rueil-Malmaison Cedex (FR)
(72) Inventeur: BADER, Jean-Marc, 69440 TALUYERS (FR); MAINTENANT, Damien, 78410 Bouafle (FR)
(74) Mandataire: IFP Energies nouvelles

(56) Documents cités:
- EMAD ALI ET AL: "Temperature Control of Ethylene to Butene-1 Dimerization Reactor", INDUSTRIAL & ENGINEERING CHEMISTRY RESEARCH., vol. 39, no. 5, 1 mai 2000 (2000-05-01), pages 1320-1329, XP055313289, US ISSN: 0888-5885, DOI: 10.1021/ie9903846
- Anurag Choudhary ET AL: "Dynamic Simulation of Reactor to Produce 1- Butene by Dimerization of Ethylene", International Journal of Scientific & Engineering Research, 1 juin 2012 (2012-06-01), XP055313294, Extrait de l'Internet: URL:http://www.ijser.org/researchpaper%5CD ynamic-Simulation-of-Reactor-to-Produce-1- Butene-by-Dimerization-of-Ethylene.pdf [extrait le 2016-10-24]

## Description

### DOMAINE DE L'INVENTION

La présente invention rentre dans le domaine des procédés de contrôle avancé (Advanced Process Control, ou « APC » selon la terminologie anglo-saxonne) et régulation des unités industrielles. Elle s'adresse plus particulièrement aux unités d'oligomérisation opérées en présence d'un catalyseur homogène en phase liquide et au point de bulle, le catalyseur étant soluble dans la phase réactionnelle. Le procédé selon l'invention s'applique préférentiellement au procédé d'oligomérisation permettant de produire du butène-1 à partir d'éthylène.

### EXAMEN DE L'ART ANTERIEUR

Le brevet EP 2 239 639 B1 décrit un procédé de contrôle et de régulation d'une unité industrielle faisant appel à une phase d'identification en boucle fermée des paramètres de fonctionnement de l'unité. Ce procédé est mis en œuvre au moyen d'un contrôleur multi-variable.
Le document publié dans INDUSTRIAL & ENGINEERING CHEMISTRY RESEARCH (vol. 39, no. 5, 1 May 2000 (2000-05-01), US, pages 1320 - 1329) décrit une méthode de contrôle de la température d'un réacteur de dimerisation d'éthylène en butène. Ce document divulgue un algorithme dit « SISO » ou « MIMO » qui prend en compte la température et la concentration en butène 1.
Le document publié dans International Journal of Scientific & Engineering Research, (Volume 3, Issue 6, June-2012) décrit une méthode de simulation dynamique d'un réacteur de production de butène par dimérisation de l'éthylène. Ce document mentionne que la pression initiale en éthylène n'a pas d'influence sur la sélectivité en butène 1.
Dans la présente invention l'unité est modélisée par un modèle dynamique dans lequel les trois variables cibles sont le débit de production de butène, la température et la pression du réacteur, et les quatre variables d'action sont le débit de charge, le débit d'eau de refroidissement qui alimente les échangeurs de la, ou des boucle(s), de recyclage du catalyseur et les deux débits des deux constituants du catalyseur ou, ce qui revient au même, le débit d'un des constituants du catalyseur et le rapport entre le débit de ce constituant du catalyseur et celui de l'autre constituant.
Sans APC, c'est-à-dire selon l'art antérieur, le mode de régulation habituel des unités de production de butène-1 par oligomérisation d'éthylène peut varier selon les unités :
- Selon un premier mode de régulation, le ratio des débits de catalyseur T2/LC est maintenu fixe, et le débit des constituants T2 ou LC du catalyseur est variable. Le plus souvent, le débit du constituant T2 est ajusté afin de contrôler la productivité de l'unité. Dans ce mode de régulation le débit d'éthylène n'est pas modifié manuellement car il est cascadé sur le contrôle de pression du réacteur. Par cascadé on entend le fait que, contrairement à une régulation PID simple dans laquelle une variable d'action (Vact) agit directement pour maintenir une variable cible (Vcb), la variable cible (Vcb) est maintenue constante par l'intermédiaire d'une autre variable cible (Vcb2) qui elle fait l'objet d'un contrôle par une autre variable d'action ( Vact2).
- Selon un second mode de régulation le ratio des débits de catalyseur T2/LC est modifié afin de contrôler la production de butene-1. Le débit d'éthylène est cascadé sur le contrôle de pression du réacteur.
L'opérateur procède généralement par pas de variation de débit de chaque constituant du catalyseur, chaque pas correspondant à un pourcentage généralement compris entre 3% et 20%, de manière plus préférée compris entre 5 et 12%, afin de limiter les fluctuations de pression du réacteur.
Une variation du débit de catalyseur engendre des effets relativement rapides au niveau du réacteur de l'unité, mais le temps de séjour global dans l'unité étant supérieur à plusieurs heures, il est nécessaire d'attendre un temps compris entre 3 heures et 10 heures selon les cas pour distinguer les effets de ces variations en sortie de l'unité. Un système de contrôle avancé peut permettre de pallier à cette difficulté.

Selon les unités il peut y avoir de une à trois boucles de recyclage de catalyseur. Dans les deux modes de régulation décrit ci-avant, la température est maintenue constante par action sur le débit de liquide de refroidissement qui alimente le ou les échangeurs de la ou des boucles de recyclage du catalyseur. La plupart des réactions d'oligomérisation conduisent également à la formation de polymères susceptibles de se déposer dans les parties froides et notamment les échangeurs, ce qui entraine une variation du coefficient d'échange et une perte d'efficacité.

### DESCRIPTION SOMMAIRE DES FIGURES

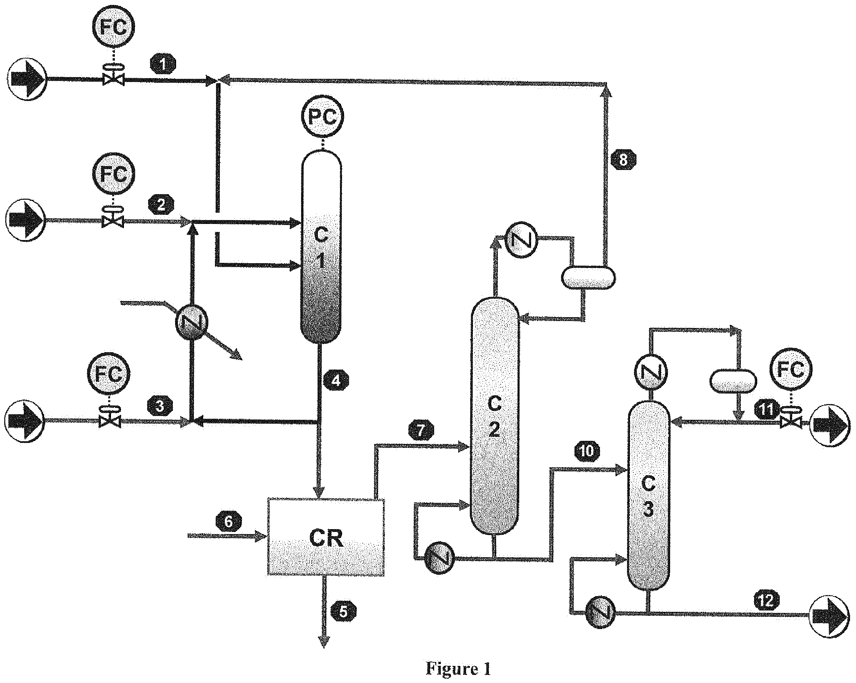

La figure 1 représente le schéma du procédé d'oligomérisation d'éthylène en butène-1 dans lequel on a fait apparaitre les principaux flux, le réacteur (C1) et les deux colonnes à distiller (C2 et C3). Sont également représentés sur ce schéma les éléments principaux du système de contrôle et régulation selon l'invention, à savoir les systèmes de contrôle ou de régulation des variables d'action (débit de charge de l'unité, débit de chaque catalyseur, débit d'eau de refroidissement) et des variables cibles (pression du réacteur, débit de produit, température du réacteur).

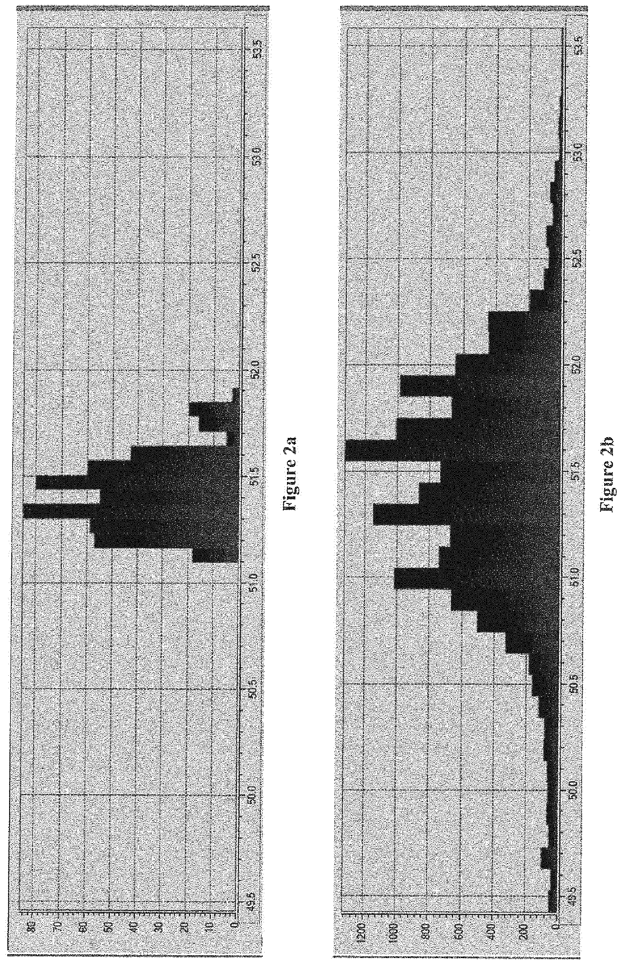

La figure 2 représente deux histogrammes de la variation de température du réacteur de l'unité en fonction du temps. La courbe de la figure 2a est selon l'invention, c'est-à-dire avec application de l'APC, et la courbe de la figure 2b est selon l'art antérieur. On constate la très nette réduction de la dispersion des valeurs avec le système de régulation APC.

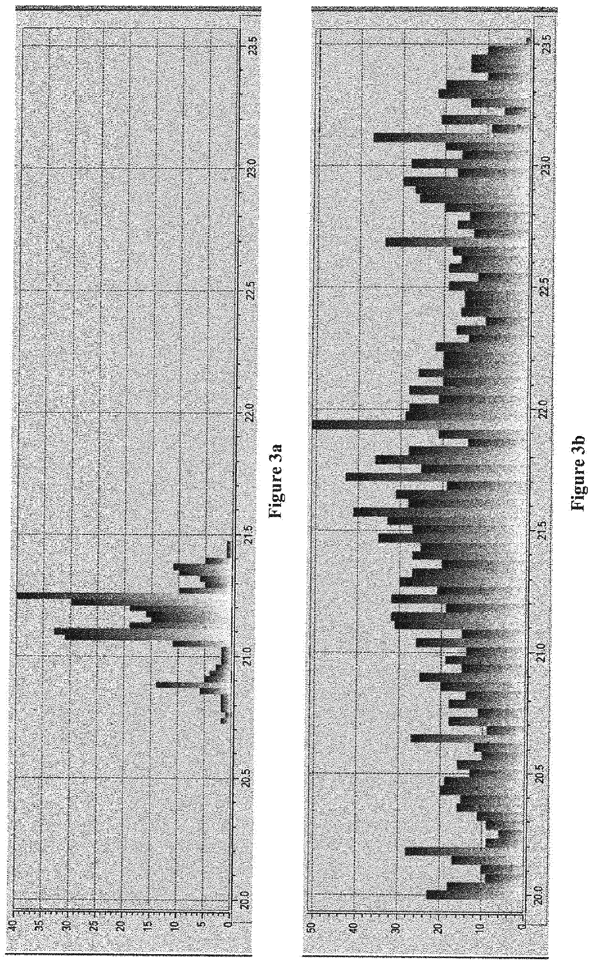

La figure 3 représente deux histogrammes de la variation de pression du réacteur au cours du temps. La courbe de la figure 3a est selon l'invention, c'est-à-dire avec application de l'APC, et la courbe de la figure 3b est selon l'art antérieur. On constate la très nette réduction de la dispersion des valeurs avec le système de régulation APC.

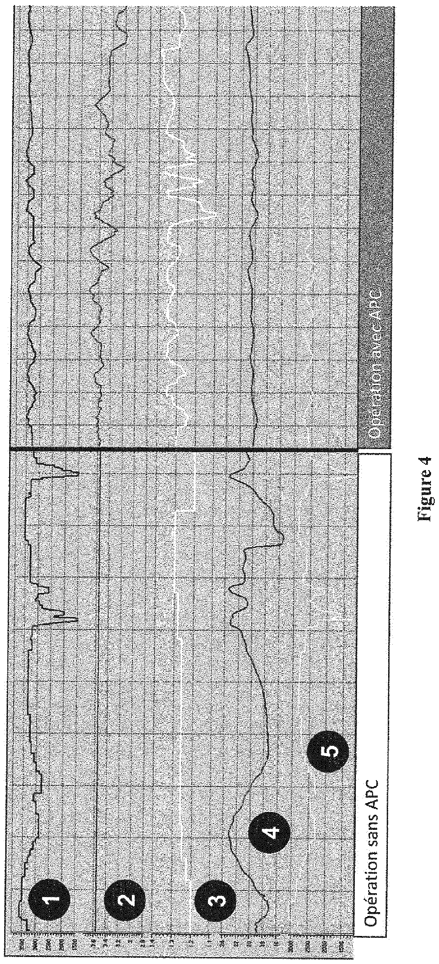

La figure 4 représente la variation dans le temps de la pression du réacteur (noté 4), et le débit de production de butene-1(noté 5), en fonction du débit de charge et du débit des deux constituants du catalyseur (notés respectivement 2 pout T2 et 3 pour LC).

Le graphique de droite est selon l'invention c'est-à-dire avec application de l'APC, et la courbe du gauche est selon l'art antérieur. Sur le graphique de droite on observe une remarquable stabilisation des deux variables cibles : la pression du réacteur et la production de butene-1.

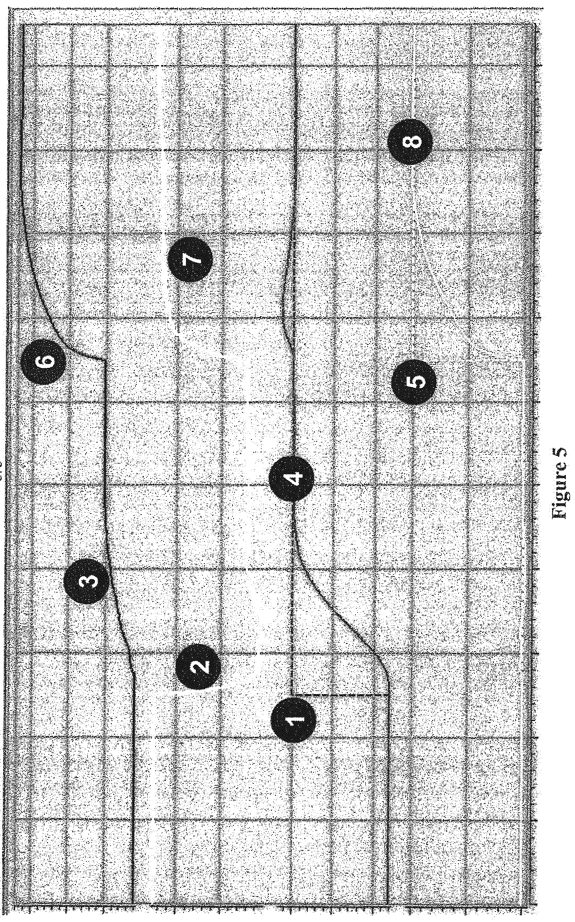

La figure 5 montre comment agissent les deux variables d'action débit de charge (noté 3 puis 6), et ratio entre les deux débits de catalyseur (noté 2 puis 7), pour opérer un changement de valeur de consigne de deux des variables cibles : débit de production de butène (noté 5 puis 8) et pression du réacteur (noté 1 puis 4).

### DESCRIPTION SOMMAIRE DE L'INVENTION

La mise en œuvre d'un procédé de contrôle avancé selon l'invention, c'est-à-dire avec APC, permet de réaliser une régulation multi-variable grâce à laquelle la température du réacteur (Treac), la pression du réacteur (Preac), et par voie de conséquence la productivité de l'unité ( Dprod) sont beaucoup plus stables.
Le procédé selon l'invention utilise un contrôleur multivariable. La modélisation dynamique du procédé de production de butène-1 par oligomérisation de l'ethylène a permis de déterminer une nouvelle stratégie de contrôle avancé, appelé dans la suite «procédé de contrôle avancé selon l'invention », qui diffère notablement des stratégies classiquement utilisées pour opérer ce type d'unité.
Selon la présente invention les 3 grandeurs que l'on cherche à maintenir constantes, dite variable cible, sont la pression du réacteur (Preac), la température du réacteur (Treac) et le débit de butène-1 produit (Dprod).
Les 4 variables d'action sur lesquelles on agit en vue de maintenir constantes les variables cibles sont : le débit d'éthylène (Dch), les débits des deux composants du catalyseur (T2 et LC) et le débit de liquide de refroidissement qui alimente le ou les échangeurs de la ou des boucles de recyclage du catalyseur.
Plus précisément, le procédé de contrôle avancé (APC) selon l'invention utilise un contrôleur multivariable (dit contrôleur APC) qui repose sur 3 principes :
- contrôle de la pression du réacteur (Preac) par la variation de la quantité de catalyseurs injectée (T2 ou LC), avec action sur le débit de charge (Dch) pour compenser les effets de la pression sur la conversion qui affecte le débit de production (Dprod),
- contrôle du débit de production (Dprod) par le débit de charge, avec action sur le débit de catalyseur (LC ou T2) pour compenser les effets sur la pression,
- contrôle de la température du réacteur (Treac) par action sur le débit de liquide de refroidissement qui alimente le ou les échangeurs de la ou des boucles de recyclage du catalyseur.

Le contrôleur APC est installé sur un calculateur, relié au système de conduite de procédé de l'unité industrielle, ledit calculateur effectuant les opérations suivantes :
- récupération des signaux des variables d'action et des variables cibles,
- génération des nouvelles consignes des variables d'action et,
   - envoi des nouvelles consignes au système de conduite de procédé.

Selon une première variante du procédé de contrôle avancé selon l'invention, on fixe le rapport des deux composants du catalyseur, T2/LC, et on agit uniquement sur le débit du composant T2. Dans ce cas, le ratio (LC/T2) est maintenu entre 1,5 et 3 moles/moles, de manière plus préférée entre 1,9 et 2,5 moles/moles, et de manière très préférée entre 2 et 2,2 moles/moles pour limiter la formation de polymères.

Selon une autre variante du procédé de contrôle avancé selon l'invention, les débits des catalyseurs T2 et LC varient librement dans une plage telle que le ratio (T2/LC) est maintenu entre 1,5 et 3 moles/moles, de manière plus préférée entre 1,9 et 2,5 moles/moles, et de manière très préférée entre 2 et 2,2 moles/moles pour limiter la formation de polymères.

Dans un mode de fonctionnement courant du procédé de contrôle avancé selon l'invention:
- Sur un changement de consigne de pression, à production constante, le débit des constituants du catalyseur est modifié afin de contrôler les variations de la pression et la modification du débit de charge compense le changement de conversion (donc de production) généré par la nouvelle consigne de pression,
- Sur un changement de consigne de production, à pression constante, le débit de charge est modifié pour contrôler la variation de la production, et la modification du débit des deux composants du catalyseur compense les effets de changement de pression générés par la nouvelle consigne de production.

Pour obtenir procédé de contrôle avancé selon la présente invention, un paramétrage dudit procédé de contrôle avancé est effectué au préalable via l'obtention de données expérimentales, ces données expérimentales provenant d'une campagne de tests consistant à générer des variations sur les variables d'action et à suivre les réponses de l'unité sur les variables cibles, variations des variables d'action et réponses des variables cibles étant traitées dans un logiciel d'identification multi-variable.

La présente invention concerne également l'application du procédé de contrôle avancé à un procédé d'oligomérisation d'éthylène pour produire du butène-1 opérant de préférence aux conditions suivantes :
- pression comprise entre 0,5 et 8 MPa, de préférence entre 1 et 4 MPa,
- température comprise entre 20°C et 150°C, de préférence entre 30°C et 100°C,

Des catalyseurs utilisables dans le procédé selon l'invention sont par exemple décrits dans les documents brevets EP-A-2388069 et EP-B-0885656 dont la description est incorporée par référence.
De manière préférée, le catalyseur comprend un premier constituant à base de titane ou de chrome, de manière plus préférée à base de titane, et un deuxième constituant à base d'aluminium.

### DESCRIPTION DETAILLEE DE L'INVENTION

La bonne compréhension de l'invention nécessite un rappel sur le fonctionnement du procédé d'oligomérisation de l'éthylène en butène-1 dans un réacteur opéré en phase liquide au point de bulle en présence d'un catalyseur homogène.
Dans une unité industrielle une fois la température et le niveau de la phase liquide dans le réacteur correctement régulés, deux autres grandeurs sont importantes à contrôler:
- La pression du réacteur, qui doit rester dans la plage de design, sous peine de perte importante de sélectivité,
- La production de butène-1, qui doit être adaptée aux besoins des unités de fabrication situées en aval.
Dans le procédé de contrôle avancé selon l'invention (avec APC), l'utilisation d'un modèle dynamique dans un contrôleur multi-variable prédictif permet de mettre en œuvre la stratégie suivante, illustrée par la simulation dynamique en boucle fermée ci-dessous :
- Contrôle de la pression par la variation de la quantité de catalyseurs injectée, avec action sur le débit de charge pour compenser les effets de la pression sur la conversion qui affecte la production.
- Contrôle de la production par le débit de charge, avec action sur la quantité de catalyseur pour compenser les effets sur la pression.
- Contrôle de la température par action sur le débit de liquide de refroidissement qui alimente le ou les échangeurs de la ou les boucles de recyclage du catalyseur.
Le contrôleur multivariable du procédé de contrôle avancé permet en sus, en cas de dépôt de polymère dans le ou les échangeurs de la boucle, d'anticiper une diminution de l'efficacité de ces échangeurs.

Un exemple de simulation selon le procédé APC de l'invention, donné ici à titre illustratif peut se résumer par les points suivants (les numéros qui apparaissent dans la description ci-dessous sont donnés en référence à la figure 5) :
- Sur un changement de consigne de pression (1), à production constante, le débit des constituants du catalyseur est modifié (2) afin de contrôler les variations de la pression.
Par ailleurs, la modification du débit de charge (3) compense le changement de conversion (donc de production) généré par la nouvelle consigne de pression (4).
- Sur un changement de consigne de production (5), à pression constante, le débit de charge est modifié (6) pour contrôler la variation de la production, et la modification du débit de catalyseur (7) compense les effets de changement de pression générés par la nouvelle consigne de production (8).
- Dans cet exemple, le contrôle de température n'est pas représenté. Toute variation de conversion affectant l'exothermicité, donc la température du réacteur, le débit d'eau de refroidissement sera ajusté en conséquence.

Grâce à l'utilisation de l'APC,
- La régulation du débit de catalyseur permet de minimiser les perturbations qui affectent la pression. La pression est stable.
- Le débit de charge et la production associée sont stables.
- La température est plus stable qu'avec les systèmes de régulations classiques, comptetenu de l'anticipation des pertes d'efficacité du ou des échangeurs.

Dans le système de régulation selon l'invention, dit système APC, on utilise le lien de proportionnalité direct qui existe entre le débit de charge en éthylène et le débit de produit en butène-1.

### DESCRIPTION DU PROCEDE DE PRODUCTION DE BUTENE-1

La figure 1 présente un schéma de principe typique d'un procédé de production de Butène-1 à partir d'éthylène.

Le procédé comprend un réacteur (C1) alimenté par une charge (1) comprenant essentiellement de l'éthylène. Le réacteur (C1) est également alimenté par un catalyseur homogène en suspension comprenant deux constituants (LC et T2) qui sont introduits séparément dans la section réactionnelle respectivement par les flux 3 et 2.

La dimérisation catalytique en phase liquide de l'éthylène est opérée à une pression comprise entre 0,5 et 8 MPa, de préférence entre 1 et 4 MPa, et une température comprise entre 20°C et 150°C, de préférence entre 30°C et 100°C.

La réaction étant exothermique, la température dans le réacteur est régulée grâce au refroidissement d'une partie de l'effluent dans la boucle comprenant un échangeur à eau (El), dont le liquide de refroidissement est de l'eau.

L'effluent de la section réactionnel (4) est ensuite mis en contact avec un inhibiteur de catalyseur dans une zone de récupération du catalyseur (CR). Tout inhibiteur de catalyseurs est utilisable dans le procédé selon l'invention, notamment ceux décrits dans le brevet FR 2 733 497. On introduit dans la zone de récupération du catalyseur un flux (4) d'inhibiteur et on récupère un flux (5) de catalyseur usé.

Une solution catalytique concentrée est séparée de l'effluent réactionnel exempt de catalyseur (7), et ledit effluent (7) est envoyé vers une section de fractionnement comprenant une première colonne (C2) qui permet de séparer en tête l'éthylène non converti (8), qui est recyclé vers la section réactionnelle, et en fond le reste de l'effluent (10) qui alimente la seconde colonne de séparation (C3).

La seconde colonne de fractionnement (C3) permet de séparer une coupe comprenant essentiellement du butène-1 (11) qui est le produit recherché des oligomères plus lourds représenté par le flux (12).

### DESCRIPTION DU PROCEDE DE CONTROLE AVANCE (APC) :

Le procédé de contrôle avancé selon l'invention, ci-après dénommé APC (pour « Advanced Process Control » selon la terminologie anglo-saxonne) peut d'une manière générale être mis en œuvre dans toute unité opérant en phase liquide et au point de bulle à partir d'une charge gazeuse, avec un catalyseur soluble dans la phase liquide (catalyseur homogène).

Dans le procédé de contrôle avancé selon l'invention, l'opérateur définit les valeurs des variables cibles, et l'APC prend en charge toutes les variables d'action sans que l'opérateur n'ait plus à s'en préoccuper. Cette stratégie permet d'obtenir une meilleure stabilité de l'unité en fonctionnement.

Le paramétrage du procédé de contrôle avancé s'effectue au préalable via l'obtention de données expérimentales. Ces données expérimentales proviennent d'une campagne de tests consistant à générer des variations sur les variables d'action. Les réponses de l'unité sur les variables cibles sont traitées dans un logiciel d'identification multi-variable. On obtient ainsi, par exemple, le délai qui correspond au temps s'écoulant entre le moment où est manipulé la variable d'action et le début de réponse de la variable cible, une constante de temps et un gain, qui définissent ensemble la dynamique d'évolution de la variable cible eu égard au changement de consigne de la variable d'action.

La température du réacteur (Treac) est contrôlée via la manipulation de contrôleurs de débit. On vérifie en la mesurant que la température de fond du réacteur est à la cible (ou à la valeur de consigne).

Les températures d'entrée des échangeurs (El) dans la boucle de recyclage du liquide comprenant le catalyseur (appelé « pumparound » dans le langage de l'homme du métier) au réacteur (C1) sont également contrôlées.

L'analyse du recycle de la phase gazeuse permet de déterminer les quantités d'éthylène et de butène-1 présentes.

La pression du réacteur (Preac) est contrôlée en jouant sur le débit des composants LC et T2 du catalyseur, ou en régulant à la fois le débit de LC et le rapport T2/LC.

La conversion par passe est généralement maintenue en dessous de 90%, de préférence en dessous de 87% pour limiter les risques de formation de polymères.

Le contrôle de la production est obtenu en jouant sur la conversion par passe et le débit d'entrée d'éthylène.

Les variables d'action permettent le contrôle simultané et continu des variables cibles, ce qui n'est pas possible sans recours à un APC, car le simple contrôle PID ne fonctionne pas correctement pour stabiliser une variable cible dépendant de multiples paramètres.

De plus, le débit de Butene-1 produit (Dprod) est proportionnel au débit entrant de charge en éthylène, et la manipulation de ce dernier pour contrôler la pression du réacteur (Preac) s'oppose donc au contrôle du débit de produit (Dprod). Seule l'utilisation du contrôle multivariable APC permet un contrôle simultané et continu de toutes les variables cibles.

### EXEMPLES SELON L'INVENTION

Une unité industrielle de production de butène-1 par oligomérisation de l'éthylène selon le schéma de la figure 1 est équipée du système de contrôle avancé selon l'invention avec les contrôleurs mentionnés dans la description et sur la figure 1.
En opérant le réacteur à 2,1 MPa et 52°C, avec le premier constituant du catalyseur LC2253 commercialisé par la société AXENS et le deuxième constituant du catalyseur TEA (TriEthylAluminium (C2H5)₃Al, dilué dans du n-hexane), l'unité produit 3,0 t/h de Butene-1. Ce ratio est de préférence maintenu entre 1,5 et 3 moles/moles, de manière plus préférée entre 1,9 et 2,5 moles/moles et de manière très préférées entre 2 et 2,2 moles/moles pour limiter la formation de polymères.
Le contrôleur APC est installé sur un calculateur, relié au système de conduite de procédé de l'unité industrielle. Le calculateur récupère les signaux des variables d'action et des variables cibles, génère les nouvelles consignes des variables d'action et les envoie au système de conduite de procédé.

La figure 2 montre que le système de contrôle avancé permet de réguler très efficacement la température de fond du réacteur avec des variations de cette température de fond 4 fois plus faibles en présence du contrôle avancé par rapport à une unité ne disposant pas de contrôle avancé.

La figure 3 montre une très bonne régulation de la pression du réacteur avec une réduction remarquable de la plage de variation de la valeur de cette pression au cours du temps, puisque la variation autour de la valeur cible est réduite d'un facteur 6 par rapport à ce qu'elle était avec le système de régulation selon l'art antérieur.

La figure 4 représente la variation dans le temps de la pression du réacteur et le débit de production de butene-1 en fonction du débit de charge et du débit des deux catalyseurs.
Le graphique de droite est selon l'invention, c'est-à-dire avec application de l'APC, et la courbe du gauche est selon l'art antérieur.
- Avec le système sans régulation APC, on constate que l'opérateur manipule uniquement le débit de charge, très peu les débits des catalyseurs. Le débit de catalyseur 1 reste même constant. Il doit réaliser un compromis entre le contrôle du débit de production et celui de la pression du réacteur.
- Avec le système de régulation APC, on constate que les débits de charge et des deux catalyseurs sont manipulés simultanément et continument pour contrôler le débit de production et celui de la pression du réacteur. On constate également, et c'est tout l'intérêt du système selon l'invention, que le débit de production et la pression du réacteur sont effectivement beaucoup mieux contrôlés, c'est-à-dire maintenus chacun au plus près de la valeur de consigne.

La figure 5 montre comment agissent deux des variables d'action; le débit de charge (noté 3 puis 6), et le ratio entre les deux débits de catalyseur (noté 2 puis 7), pour opérer un changement de valeur de consigne sur deux des variables cibles : débit de production de butène (noté 5 puis 8) et pression du réacteur (noté 1 puis 4).
Les deux changements sur deux des variables cibles, à savoir la production en butène-1 et la pression du réacteur, sont pris en charge :
- par la variable débit de charge dans un premier temps par une montée très lente (repérée 3), puis dans un second temps par une montée plus rapide (repérée 6),
- par la variable ratio des débits de catalyseur dans un premier temps par une baisse de cette valeur (repérée 2), puis dans un second temps par une remontée de la valeur sensiblement au même niveau que la valeur de départ (repérée 7).

## Revendications

1. Procédé de contrôle avancé (APC) applicable aux unités d'oligomérisation de l'éthylène en butène-1 opérées dans un réacteur en présence d'un catalyseur homogène en phase liquide et au point de bulle, **caractérisé en ce que** les grandeurs contrôlées simultanément en continu, dites grandeur cibles, sont la production de butène-1 (Dprod), la pression du réacteur (Preac), et la température du réacteur (Treac), et les variables d'action sont le débit de charge (Dch), le débit de chacun des composants du catalyseur injecté savoir T2 à base de titane et LC à base d' aluminium, et le débit de liquide de refroidissement qui alimente le ou les échangeurs de la ou des boucles de recyclage du catalyseur, le contrôleur multivariable du procédé de contrôle avancé, dit contrôleur APC, agissant de la façon suivante :
- contrôle de la pression du réacteur (Preac) par la variation de la quantité de catalyseur injectée, avec action sur le débit de charge pour compenser les effets de la pression sur la conversion qui affecte la production,
- contrôle de la production (Dprod) par le débit de charge, avec action sur la quantité de catalyseur pour compenser les effets sur la pression,
- contrôle de la température du réacteur (Treac) par action sur le débit de liquide de refroidissement qui alimente le ou les échangeurs de la ou des boucles de recyclage du catalyseur,
le contrôleur APC étant installé sur un calculateur, ledit calculateur effectuant les opérations suivantes :
- récupération des signaux des variables d'action et des variables cibles,
- génération des nouvelles consignes des variables d'action et,
- envoi des nouvelles consignes au contrôleur APC.

2. Procédé de contrôle avancé selon la revendication 1, dans lequel les débits des composants du catalyseur, à savoir T2 à base de titane et LC à base d'aluminium, varient librement dans une plage telle que le ratio (T2/LC) est maintenu entre 1,5 et 3 moles/moles, de manière préférée entre 1,9 et 2,5 moles/moles, et de manière très préférée entre 2 et 2,2 moles/moles pour limiter la formation de polymères.

3. Procédé de contrôle avancé selon la revendication 1, dans lequel :
- Sur un changement de consigne de pression, à production constante, le débit des constituants du catalyseur est modifié afin de contrôler les variations de la pression et la modification du débit de charge compense le changement de conversion, donc de production de butène-1, généré par la nouvelle consigne de pression,
- Sur un changement de consigne de production, à pression constante, le débit de charge est modifié pour contrôler la variation de la production, c'est-à-dire le débit de butène-1, et la modification du débit des deux composants du catalyseur compense les effets de changement de pression générés par la nouvelle consigne de production.

4. Procédé de contrôle avancé selon la revendication 1, dans lequel le paramétrage du procédé de contrôle avancé s'effectue au préalable via l'obtention de données expérimentales, ces données expérimentales provenant d'une campagne de tests consistant à générer des variations sur les variables d'action et les réponses de l'unité sur les variables cibles étant traitées dans un logiciel d'identification multi-variable.

5. Application du procédé de contrôle avancé selon l'une des revendications 1 à 4, à un procédé d'oligomérisation d'éthylène pour produire du butène-1 opérant aux conditions suivantes :
- pression comprise entre 0,5 et 8 MPa, de préférence entre 1 et 4 MPa,
- température comprise entre 20°C et 150°C, de préférence entre 30°C et 100°C.

## Patentansprüche

1. Erweiterte Prozessteuerung (Advanced Control Process (APC)), die bei Einheiten zur Oligomerisierung von Ethylen zu Buten-1 eingesetzt werden kann, die in einem Reaktor in Gegenwart eines homogenen Katalysators in flüssiger Phase und am Blasenpunkt betrieben werden, **dadurch gekennzeichnet, dass** die gleichzeitig kontinuierlich gesteuerten Größen, genannt Zielgrößen, die Produktion von Buten-1 (Dprod), der Druck des Reaktors (Preac) und die Temperatur des Reaktors (Treac) sind, und die Aktionsvariablen die Beschickungsrate (Dch), die Durchflussrate jeder der Komponenten des eingespritzten Katalysators, nämlich T2 auf Basis von Titan und LC auf Basis von Aluminium, und die Durchflussrate des Kühlmittels, das den oder die Tauscher der Katalysatorrecyclingschleife(n) speist, sind, wobei die Steuerung mit mehreren Variablen der erweiterten Prozessteuerung, genannt APC-Steuerung, auf folgende Weise arbeitet:
- Steuern des Reaktordrucks (Preac) durch Variieren der Menge der eingespritzten Katalysatoren mittels Beeinflussung der Beschickungsrate, um die Auswirkungen des Drucks auf die Umwandlung auszugleichen, der die Produktion beeinflusst,
- Steuern der Produktion (Dprod) durch die Beschickungsrate mittels Beeinflussung der Katalysatormenge, um die Auswirkungen auf den Druck auszugleichen,
- Steuern der Reaktortemperatur (Treac) durch Beeinflussung der Durchflussrate des Kühlmittels, das den oder die Tauscher der Katalysatorrecyclingschleife(n) speist,
wobei die APC-Steuerung auf einem Computer eingerichtet ist, wobei der Computer die folgenden Arbeitsgänge ausführt:
- Abrufen der Signale der Aktionsvariablen und der Zielvariablen,
- Erzeugen neuer Sollwerte der Aktionsvariablen und
- Senden der neuen Sollwerte an die APC-Steuerung.

2. Erweiterte Prozessteuerung nach Anspruch 1, wobei die Durchflussraten der Komponenten des Katalysators, nämlich T2 auf Basis von Titan und LC auf Basis von Aluminium, so in einem Bereich frei variieren, dass das Verhältnis (T2/LC) zwischen 1,5 und 3 Mol/Mol, vorzugsweise zwischen 1,9 und 2,5 Mol/Mol und stärker bevorzugt zwischen 2 und 2,2 Mol/Mol gehalten wird, um die Bildung von Polymeren zu begrenzen.

3. Erweiterte Prozessteuerung nach Anspruch 1, wobei:
- bei einer Änderung des Drucksollwerts bei konstanter Produktion die Durchflussrate der Katalysatorkomponenten modifiziert wird, um die Druckschwankungen zu steuern, und die Modifikation der Beschickungsrate die Änderung der Umwandlung, also der Produktion von Buten-1, ausgleicht, die durch den neuen Drucksollwert erzeugt wird,
- bei einer Änderung des Produktionssollwerts bei konstantem Druck die Beschickungsrate modifiziert wird, um die Produktionsschwankungen zu steuern, das heißt die Durchflussrate von Buten-1, und die Modifikation der Durchflussrate der beiden Katalysatorkomponenten die Auswirkungen von Änderungen des Drucks ausgleicht, die durch den neuen Produktionssollwert erzeugt werden.

4. Erweiterte Prozessteuerung nach Anspruch 1, wobei die Konfiguration der erweiterten Prozessteuerung vorher durch Erhalten von Versuchsdaten durchgeführt wird, wobei diese Versuchsdaten aus einer Testkampagne stammen, die darin besteht, Schwankungen der Aktionsvariablen zu erzeugen, und den Antworten der Einheit auf die Zielvariablen, die in einer Software zur Identifizierung mehrerer Variablen verarbeitet werden.

5. Anwendung der erweiterten Prozesssteuerung nach einem der Ansprüche 1 bis 4 auf ein Verfahren zur Oligomerisierung von Ethylen zur Produktion von Buten-1, das unter den folgenden Bedingungen arbeitet:
- einem Druck zwischen 0,5 und 8 MPa, vorzugsweise zwischen 1 und 4 MPa,
- einer Temperatur zwischen 20 °C und 150 °C, vorzugsweise zwischen 30 °C und 100 °C.

## Claims

1. Advanced process control (APC) process which can be applied to units for the oligomerization of ethylene to 1-butene which are operated in a reactor in the presence of a homogeneous catalyst in the liquid phase and at the bubble point, **characterized in that** the parameters which are simultaneously and continuously controlled, termed the target parameters, are the production of 1-butene (Dprod), the reactor pressure (Preac) and the reactor temperature (Treac), and the action variables are the feed flow rate (Dch), the flow rate of each of the components of the injected catalyst, namely T2 based on titanium and LC based on aluminium, and the flow rate of cooling liquid which is supplied to the exchanger or exchangers of the catalyst recycling loop or loops, with the multivariable controller of the advanced process control process, termed the APC controller, acting as follows:
- controlling the reactor pressure (Preac) by varying the quantity of catalyst injected, by acting on the feed flow rate in order to compensate for the effects of pressure on the conversion which affects the production,
- controlling the production (Dprod) by the feed flow rate, by acting on the quantity of catalyst in order to compensate for the effects on the pressure,
- controlling the reactor temperature (Treac) by acting on the flow rate of cooling liquid which is supplied to the exchanger or exchangers of the catalyst recycling loop or loops,
the APC controller being installed in a computer, said computer carrying out the following operations:
- recovering signals for the action variables and the target variables,
- generating new setpoints for the action variables, and
- sending the new setpoints to the APC controller.

2. Advanced process control process according to Claim 1, in which the flow rates of the catalyst components, namely T2 based on titanium and LC based on aluminium, vary freely within a range such that the ratio (T2/LC) is kept between 1.5 and 3 moles/moles, preferably between 1.9 and 2.5 moles/moles, and very preferably between 2 and 2.2 moles/moles in order to limit the formation of polymers.

3. Advanced process control process according to Claim 1, in which:
- upon a change in the pressure setpoint, at constant production, the flow rate of the constituents of the catalyst is modified in order to control the variations in the pressure, and the modification of the feed flow rate compensates for the change in conversion, and thus in 1-butene production, generated by the new pressure setpoint,
- upon a change in the production setpoint, at constant pressure, the feed flow rate is modified in order to control the variation in production, i.e. the flow rate of 1-butene, and the modification of the flow rate of the two components of the catalyst compensates for the effects of the change of pressure generated by the new production setpoint.

4. Advanced process control process according to Claim 1, in which parameterization of the advanced process control process is carried out in advance by obtaining experimental data, these experimental data deriving from a campaign of tests consisting of generating variations in the action variables, and the responses of the unit on the target variables being processed in multivariable identification software.

5. Application of the advanced process control process according to one of Claims 1 to 4 to a process for the oligomerization of ethylene in order to produce 1-butene, operating under the following conditions:
- pressure of between 0.5 and 8 MPa, preferably between 1 and 4 MPa,
- temperature of between 20°C and 150°C, preferably between 30°C and 100°C.
